(19) Europäisches Patentamt
European Patent Office
Office européen des brevets

(11) Publication number: **0 133 905 B1**

(12) # EUROPEAN PATENT SPECIFICATION

(45) Date of publication of patent specification: **18.09.91** (51) Int. Cl.5: **A61K 7/06**

(21) Application number: **84107545.0**

(22) Date of filing: **29.06.84**

(54) **Low pH hair conditioner and neutralizer conditioning compositions containing amine oxides.**

(30) Priority: **30.06.83 US 509570**

(43) Date of publication of application:
**13.03.85 Bulletin 85/11**

(45) Publication of the grant of the patent:
**18.09.91 Bulletin 91/38**

(84) Designated Contracting States:
**AT BE CH DE FR GB IT LI LU NL SE**

(56) References cited:
**EP-A- 0 082 299**
**FR-A- 2 190 406**
**FR-A- 2 358 877**
**FR-A- 2 381 813**
**US-A- 3 926 861**

(73) Proprietor: **Revlon, Inc.**
**767 Fifth Avenue**
**New York, N.Y.10022(US)**

(72) Inventor: **Gerstein, Terry**
**1944 Lowell Lane**
**Merrick, N.Y. 11566(US)**
Inventor: **Goldberg, Marvin**
**10 Dana Road**
**Monsey, N.Y. 10952(US)**

(74) Representative: **Körber, Wolfhart, Dr.rer.nat. et al**
**Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10**
**W-8000 München 22(DE)**

**EP 0 133 905 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to hair conditioning compositions. More particularly, this invention relates to aqueous hair conditioning compositions that comprise relatively high molecular weight amine oxides at low pH's and to neutralizer conditions.

After washing hair and during the subsequent management of the dry hair, the combing and brushing forces produce friction resulting in the accumulation on the hair's surface of immobile electrons or ions. The hair is commonly referred to as containing static charge and displays the phenomenon of fly away. Such hair is unruly, will not lay flat and is considered generally unmanageable.

Hair treatment products must be capable of leaving the hair with luster and softness while overcoming natural or artificially induced dryness and roughness produced by the actions of the environment, such as sunlight, and cosmetic products, such as products for permanent waving and dyeing. It is also important that such hair treating products be capable of restoring the hair as nearly as possible to its original state in relation to the feel of the hair, and ease of combing.

Hair conditioning agents assist in the control and management of hair. Conditioned hair is easily untangled and combed through after shampooing, lays orderly when dry and provides a favorable feeling to the touch. The conditioning action on hair, particularly by cationic conditioning agents, is believed to be caused by the attraction of the positively charged agent to the negative sites on hair protein resulting in the deposition of the agent on to the hair fiber.

Ionic depositions including positively charged cationic conditioning agents can be used to dissipate static electricity by increasing the mobility of the electrostatic charges that accumulate on hair. Furthermore, the fatty nature of the cationic conditioning agent produces lubrication on the hair's surface that reduce the frictional forces, resulting in both the overall lessening of accumulated electrostatic charges and easy combing. The process by which cationic surfactants are applied to hair is referred to as conditioning the hair and the treatment results in hair that no longer sustains a static charge when dry and is readily combed through when wet. Conditioned hair also feels soft, silky and is highly manageable.

Oxidizing agents, such as hydrogen peroxide, used for hair bleaching and coloring are normally employed at alkaline pHs of about 9.5 or greater. At such alkaline pHs both the bleaching and reactivity, to produce hair color from the relatively colorless dye intermediates, of the oxidizing agents are maximized. At acid pHs the reactivity of oxidizing agents is extremely slow, producing impractically low hair bleaching and coloring activity.

In addition to their use for bleaching and coloring hair at alkaline pHs, oxidizing agents are used to restore sulphur bonds within hair and to deactivate residual reducing agents left on hair from chemical treatment, such as treatment with a hair straightening or waving preparation. The application of a permanent waving lotion, the active ingredient of which is a mercaptan reducing agent, reduces or breaks the disulfide cystine amino acid bonds of the hair structure and makes the hair pliable, soft and weak so that the hair can be reformed to another configuration. If no subsequent treatment is done to the hair, oxygen from the atmosphere could slowly act to restore the sulphur bonds. However, this process requires a long time to be effective during which additional physical damage may occur to the hair thereby limiting the user in her activities and convenience. In order to quickly restore the strength of hair and to fix the new configuration of the hair, the disulfide bonds must be re-established by the use of an acidic oxidative preparation. The action of an oxidative agent, typically hydrogen peroxide, in the acidic preparation, provides an immediate and effective result in reconstructing the disulfide bonds. Both the acidity of the preparation and the presence of hydrogen peroxide serve to quickly restore the hair to its normal condition providing close to normal tensile strength and reducing the chance of accidental damage to it in its weakened state.

In addition to the foregoing advantages, an acidic oxidative preparation also rids the hair of trace alkali and/or reducing agents which tend to be left on the hair from hair straighteners and permanent waving products. This action of the acidic oxidative preparations is also essential because the chemical action of alkalis on hair keratin continues long after their application is completed. Furthermore, both sulphur reducing chemicals, such as the salts and esters of thioglycolic acid, sulfite and bisulfite salts, and unrestored disulfide cystine bonds in hair keratin are the source of unpleasant mercaptan odors that persist on hair after treatment. These odors render the hair offensive. Treatment with an acidic hydrogen peroxide preparation, therefore, also serves to deodorize the hair thereby providing aesthetic properties. Even further, the oxidative hair preparation also serves as a mild antiseptic to scalp promoting scalp hygiene.

Notwithstanding the advantages offered by oxidative hair preparations, the hair treated therewith lacks the desired texture, it is raspy, troublesome to comb, and requires further conditioning. To correct these conditions some manufacturers of hair treatment products frequently supply post-treatment conditions usually as part of a kit included with the acidic oxidative preparation. Others incorporate specific hair

conditioning agents compatible with the oxidizing agents, such as nonionic and cationic agents.

Cationic surfactants have been used extensively as hair conditioning agents in creme rinses and shampoos, generally at pH levels below pH 7 in creme rinses and through pH 7 and above in shampoos. In the past best results in creme rinses have been obtained with cationic surfactants that are long chain high molecular weight quaternary ammonium compounds or long chain fatty amine salts. For example, stearalkonium chloride has been widely used as a component of creme rinse hair conditioning formulations. The positive charge of the quaternary surfactant is attracted to the negatively charged surface of the hair protein deposits on the surface and subsequently renders the hair manageable. The long chain constituent on the quaternary surfactant coats the hair fiber giving it lubricity during wet combing and a desirable texture after drying. The longer the chain length then the more active the conditioning agent is said to be; the greater the residual film deposit on hair then the easier the detangling effort and the less electrostatic charge build-up and subsequent hair flyaway.

Quaternary ammonium compounds carry and maintain positive ionic charges from highly alkaline to highly acidic media. However, many industrial quaternary ammonium compounds are partially or totally unsuitable for cosmetic use because they can contain impurities which restrict use to specific pH ranges or restrict use completely. If trace quantities of deleterious quaternizing agents used in synthesis are present, the quaternary ammonium compound should not be used in cosmetics. Long chain fatty amines, which usually account as significant impurity in the quaternary ammonium compounds used for cosmetics, limit the use of the quaternary ammonium compound, and the cosmetic itself, to pHs, below 7. Below pH 7 the long chain fatty amines exist as surface active salts which produce similar hair conditioning effects as surface active quaternary ammonium compounds. Above pH 7 the amine salts revert to their free organic amine state which cause them to become more water insoluble and loose their surfactant properties, to loose their hair conditioning properties, to produce cosmetically unaesthetic odors and appearances, and to increase irritation to the skin and eyes.

On the other hand, long chain amine oxide surfactants have been freely used in acidic and alkaline cosmetics, toiletries and other consumer products. In alkaline products amine oxide surfactants behave as nonionics; they bear neither positive or negative molecular charges. The nonionic character allows them to be compatible with anionic ingredients in shampoos. Here, amine oxides can serve as foam contributors, foam stabilizers, viscosity enhancing agents, super fatting agents, etc. In nonionic and anionic hair conditioners they can be employed as emulsifiers, thickeners and used for complementing typical fats, oils and waxes. The diversity of use of amine oxide surfactants are exemplified by the following disclosures.

In U.S Patent No. 3,990,991 aqueous shampoo-conditioner formulations are described comprising amphoteric, cryptoanionic and cationic surfactants. Amine oxides having surfactant properties are mentioned as useful cationic surfactants, although in the specific examples preferred quaternary ammonium compounds are employed in combination with amine oxide; the pH of each such formulation is above 6.

In U.S. Patent No. 4,007,261 conditioner compositions are described which consist essentially of an aqueous emulsion of an alkyl dimethyl amine oxide having from 16 to 22 carbon atoms in the alkyl chain, the pH of which compositions are preferably adjusted to between about 5.0 and 6.0. Specifically the invention encompasses pearlescent effects in such hair conditioners. However, it was found that such amine oxide formulations, namely the formulations having pH values described in this patent, did not provide adequate hair conditioning properties relative to quaternary ammonium surfactants. Augmentation of the amine oxide with quaternary ammonium was required to impart good conditioning properties to formulations. See also, D&CI, July 1981, pgs. 40-42, "Amine Oxides in Cosmetic Formulations", Klein, where "slightly acid pH" amine oxides were suggested as contributing to hair manageability.

U.S. Patent No. 4,229,313 employs amine oxides as viscosity builders in cleaning and bleaching compositions for fabrics; both U.S. Patent Nos. 4,048,338 and 4,033,895 use amine oxides as counter irritant ingredients in toiletry products to render them milder; U.S. Patent No. 4,179,504 uses amine oxides as pharmacologically active ingredients in insecticidal and ovacidal preparations; U.S. Patent No. 4,325,821 incorporates amine oxides into an improved froth flotation process to separate mineral ores; German Patent No. 2,748,463 employs amine oxides as solubilizing agents for vitamine B derivatives in antiseborrheic products.

FR-A-2,190,406 relates to hair treating compositions for bleaching, waving or straightening, or coloring or dying hair which contain high molecular weight water soluble polymers having a multiplicity of secondary amino or of quaternary salt groups. A surface active agent is present as an essential ingredient of this hair coloring or hair bleaching composition. The amine oxide mentioned is dimethyloctadecylamine oxide.

However, its function in the various compositions disclosed in the FR-A-2,190,406 is that of a surfactant only.

FR-A-2,381,813 relates to improved foam compositions for body and hair care. Some of these

compositions contain from 0.2 to 3% of a tertiary amino oxide; alkyldimethylamino oxides being specifically mentioned. The above compositions comprise a propellant, at least one amphoteric tensio-active agent, and a tertiary amino oxide. The object of this invention is to provide a foam of increased stability and greater foam production.

Obviously, the tertiary amino oxide is employed in combination with the tensio-active amphoteric agent because of its foam producing characteristics in aerosol applications.

In the US-A-3,926,861 morpholine oxides and their use in detergent compositions are described. The attributes of said morpholine oxides are a desirable effect on the skin, and particularly a non-irritating and even anti-irritating effect when used in conjunction with surface active agents that ordinarily irritate the skin. Said compounds may be used in hair shampooing hair-dying, or other hair-treating or hair-conditioning compositions, i.e., they have a desirable effect on the skin, or by inference, on the scalp, and their attributes of particular value are in emulsification and cleansing and detergency. The desirable effect of morpholine oxides on the skin in the US-A-3,926,861 is particularly beneficial when used in combination with other, more irritating surface active agents.

The EP-A-0082299 describes hair shampooing compositions comprising triamine trioxides which are utilized in the pH control through the use of acids.

In acid media nonionic amine oxide surfactants acquire a positive charge through the inductive effects of hydrogen ions of the media. The amine oxides can behave cationic, however weakly cationic, since the positive charge produces less ionicity than that of quaternary ammonium salts or fatty amine salts. The positive charge, nevertheless, permits complete compatibility in cationic preparations and allows the cationicity of the amine oxide to support the cationicity of the dominant surfactant.

An examination of the ingredient content of popular creme rinses, instant conditioners and balsams for hair sold on the market show that these products do not rely solely on amine oxides. These acidic preparations employ quaternary ammonium surfactants and/or fatty amine salts, which are both strongly cationic to product hair conditioning. If amine oxides are present in the ingredient content, they are used for producing conditioning ancillary to the primary cationic surfactant as well as for other specific properties such as thickening, foaming emulsification, etc. Amine oxide surfactants are recognized as not adsorbing to hair as strongly as quaternary ammonium surfactants and, therefore, do not produce as intense hair conditioning effects as quaternary ammonium salts.

Although it was known that amine oxides develop cationic properties, in other words, a net positive charge, at low pH values for the reasons stated above, it has not been recognized that the cationic properties of amine oxides could be solely usefully employed to condition hair at low pH values.

The present invention relates to a neutralizer conditioning composition which includes 0.1 to 10% by weight of a cosmetically acceptable oxidizing agent, water, sufficient acid to provide a pH for the composition between 4.5 and 1.8, and an amine oxide acquiring a sufficient positive charge to allow the amine oxide to be substantive onto the hair, said amine oxide having a concentration from 0.1% to 10% of the total weight of the neutralizer conditioning composition, the amine oxide having a general formula selected from:

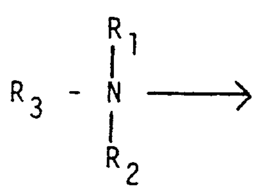

(I)

wherein $R_1$ is lower ($C_1$-$C_4$) alkyl, alkoxyalkyl or hydroxyalkyl, $R_2$ is lower to long chain ($C_1$-$C_{22}$) aliphatic hydrocarbyl radical, alkoxyalkyl or hydroxyalkyl, and $R_3$ is aliphatic hydrocarbyl moiety of 8 to 22 carbon atoms;

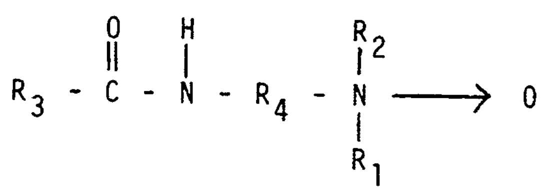

(II)

wherein $R_1$ and $R_2$ are the same or different moieties and are selected from lower ($C_1$-$C_4$) alkyl, alkoxyalkyl or hydroxyalkyl groups, $R_3$ is a moiety containing an alkyl chain of 8 to 22 carbon atoms, and $R_4$ is a moiety selected from lower ($C_1$-$C_4$) alkylene groups;

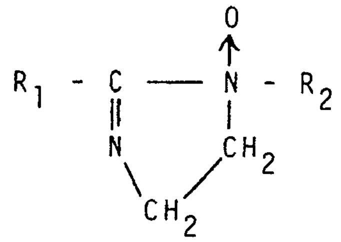

(III)

wherein $R_1$ is a moiety having an alkyl chain of 8 to 22 carbon atoms, and $R_2$ is a moiety selected from 2-hydroxyethyl, a derivative of 2-hydroxyethyl, and a nonionic derivative of 2-amino ethyl;

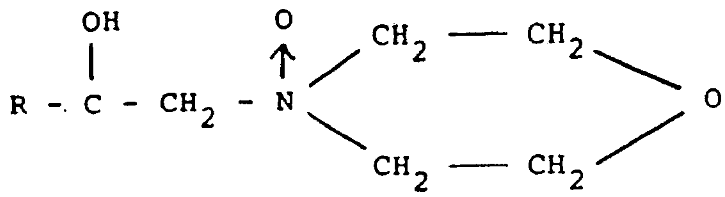

(IV)

wherein R is a moiety having an alkyl chain of 6 to 17 carbon atoms; or

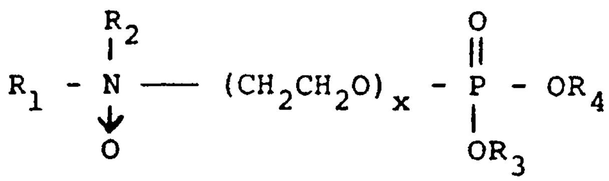

(V)

wherein $R_1$ is a moiety that contains an alkyl chain having 8 to 22 carbon atoms, $R_2$ is a moiety selected from

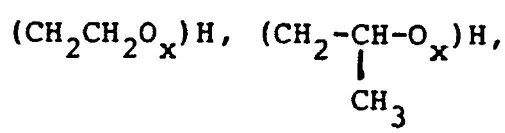

and an alkyl chain of 8 to 22 carbon atoms, x is an integer from 1 to 30, and $R_3$ and $R_4$ are the same or different moieties and are selected from a lower ($C_1$-$C_4$) alkyl group.

It has now been found that at pH values of about the isoelectric pH of hair protein, or less, the relatively high molecular weight amine oxides described herein perform advantageously as described conditioning agents for hair.

It has also been found that an excellent acidic oxidative conditioning preparation can be prepared by incorporating a new oxidizing agent with surface active amine oxide systems described herein at pH values at about the isoelectric pH of hair protein or less.

The isoelectric point of hair, the state of which the positive and negative ionic charges of hair protein becomes balanced, occurs under acid conditions. The exact point is not exactly known and investigators differ on the range of isoelectric point pH values; pH 3.3 to 4.5 by Cook and Smith, Appl. Polym. Symp. 18, 663 (1971), and more recently pH 2.45 to 3.17 by Parreira, Journal of Colloid and Interface Science, Vol. 75; No. 1 (1980). At the isoelectric point protein carries a neutral charge.

An advantage occurs from inducing hair protein to gather a neutral ionic charge, that is, to treat hair at its isoelectric point. At the isoelectric point, hair protein, keratin, displays its greatest insolubility and

greatest ionic stability against chemical reaction. Hair treated at its isoelectric point pH so that hair protein can acquire an uncharged neutral state is conceivably rendered stronger than treatment at other pH's although this is difficult to prove.

Accordingly, this invention provides a composition and means for taking advantage of the cationic properties of amine oxides which have been unapparent at low pH values.

It has been surprisingly found that the pH range on or about the isoelectric point of hair or less, amine oxides acquire a sufficient positive charge to allow them to be substantially substantive onto the hair. The adsorption is to such an extent that it can allow the employment of amine oxides as the sole conditioning agents in creme rinse products. The rationale for such a phenomena is apparently obscure since at its isoelectric point, hair protein carries no polar charges and should not particularly absorb the positive charged amine oxide molecules.

The precise mechanism is not known by which this unexpected adsorption and resulting conditioning effect at this low pH value takes place. However, with hindsight it is speculated that the mechanism allows amine oxide surfactant to replace absorbed hydrogen ions on the hair's surface. At the isoelectric point of hair, that is at approximately a pH range from pH 2.4 to 4.5 all of the available negative sites on the hair's surface filled by positively charged hydrogen ions, i.e., the hair becomes ionically neutral. It is believed that at these low pHs amine oxide molecules gather a sufficient positive charge to allow them to compete for the sites held by the positive hydrogen ions on the surface of hair. Ion exchange takes place in which the amine oxide molecules substitute for the hydrogen ions. The ion exchange is accelerated by the surface activity of the amine oxide molecule which propel the amine oxide molecules to the surface of hair. Hence, it is the combination of surface activity and a significantly acquired positive charge through induction in acidic media that allow for the unusual and unexpected effects of amine oxides at or about the isoelectric point of hair or less.

This invention also offers the advantage of employing conditioning agents that are considered milder to the user than quaternary ammonium compounds. Amine oxides have been described as non-irritating in cosmetic systems and the present invention allows the use of such conditioning agents without addition of potentially irritating quaternary ammonium salts.

Moreover, the conditioning balance of the hair conditioning composition of this invention may be easily modified by adjusting the pH of the formulation, the concentration of the amine oxide, or the physical or chemical nature of the amine oxide as governed by the properties of the long chain alkyl group(s). At low pH values, the effectiveness of amine oxides are maximized because of a greater proportion of the amine oxide molecules are converted to their cationic form. This enables reduced quantities of amine oxides to be used. Or, if less conditioning is desired, the pH of the compositions may be increased and/or the concentration of the amine oxides may be increased and/or the concentrations of the amine oxides may be reduced. One skilled in this art will readily understand how to achieve a balance between pH and amine oxide concentration for one's conditioning requirement.

It has also been surprisingly found that oxidizing agents may be incorporated into the herein described conditioning preparation, thereby obtaining a composition that functions both as neutralizer and as a conditioner. Oxidizing agents, such as hydrogen peroxide are used in neutralizing preparations to oxidize the sulfhydryl groups formed in hair by the reducing agents of chemical hair treatments. They help restore the tensile strength to hair is removed by chemical processing. Such a composition, as contrasted with conventional neutralizers having the previously described disadvantages, provide as a result of treatment therewith aesthetically pleasing, smooth, easy-to-comb hair with full body, and without the static fly-away characteristics.

Amine oxides are the N-oxides of tertiary amines. They may be prepared by methods well known in the art, for example, by reaction of the desired tertiary amine with hydrogen peroxide.

One example of the amine oxides which are useful in preparing the conditioning composition of this invention include compounds represented by Formula I.

Examples of suitable amine oxides of Formula I which are useful in this invention include lauryl dimethyl amine oxide, ($C_{14}H_{29}$) dimethyl amine oxide, cetyl dimethyl amine oxide, ($C_{18}H_{37}$) dimethyl amine oxide, ($C_{18}H_{35}$) dimethyl amine oxide, heptadecyl dimethyl amine oxide, ($C_{22}H_{45}$) dimethyl amine oxide, dimethyl ($C_{18}$) hydrocarbon amine oxides including mixtures of octadecadienyl, octadecadicenyl and octadecadecyl amine oxides, dimethyl ($C_{17}$-$c_{19}$) amine oxides, bis (hydroxyethyl) ($C_{18}$) hydrocarbon amine oxides including mixtures of octadecadienyl, octadecadicenyl and octadecadecyl amine oxides, bis (hydroxyethyl) tallow amine oxide, bis (hydroxypropyl) ($C_{17}H_{35}$) amine oxide, bis (hydroxymethyl) ($C_{21}H_{43}$) amine oxide, pentadecyl diethyl amine oxide, tridecyl dipropyl amine oxide, tridecyl (2-hydroxybutyl) amine oxide, heptadecyl bis (2-hydroxybutyl) amine oxide, tridecyloxypropyl bis (hydroxyethyl) amine oxide.

Generally, amine oxides having major alkyl chains of less than about 8 carbon atoms do not provide

adequate hair conditioning and tend to be irritating to the user. Better conditioning results are obtained with single long chain amine oxides having 12 or more, and preferably 14-22 carbon atoms in the long chain group and with double long chain amine oxides having as few as 8 carbons in their long chain groups. Preferred among the amine oxides is stearyl dimethyl amine oxide.

Further examples of suitable amine oxides of Formula I which are useful in this invention include di($C_{18}$) hydrocarbon methyl amine oxides, di($C_{18}H_{37}$) methyl amine oxide, di($C_{17}$-$C_{19}$) methyl amine oxide, dicetyl methyl amine oxide, cetyl isocetyl methyl amine oxide, lauryl cetyl methyl amine oxide, di($C_{18}H_{33}$) methyl amine oxide, di($C_{18}H_{35}$) methyl amine oxide, diiso($C_{18}H_{37}$) methyl amine oxide, di($C_{18}H_{37}$) hydroxyethyl amine oxide, ($C_{18}H_{37}$) hydrocarbons , iso($C_{18}H_{37}$) hydroxymethyl amine oxide, hexyl bis (2-hydroxyhexadecyl) amine oxide and di($C_{18}H_{37}$) monomethyl amine oxide.

Examples of suitable amine oxides represented by Formula II, which are useful in this invention, include cocoylamidopropyl dimethyl amine oxide, myristoylamidopropyl dimethyl amine oxide, stearoylamidoethyl dimethyl amine oxide, linoleoylamidopropyl dimethyl amine oxide, hydrogenated tallow amidoethyl bis (hydroxyethyl) amine oxide, palmitoylamidoethyl bis (hydroxypropyl) amine oxide, stearoylamidopropyl dimethyl amine oxide, and hydrogenated tallow amidopropyl dimethyl amine oxide.

Examples of suitable amine oxides represented by Formula III, which are useful in this invention, include oleyl imidazoline [(oleic) 1-hydroxyethyl-2-heptadecenyl-2-imidazoline-1-oxide], (stearic) 1-hydroxyethyl-2-heptadecanyl-2-imidazoline-1-oxide, 1-acetylhydroxyethyl-2-tridecanyl-2-imidazoline-1-oxide; 1-acetylaminoethyl-2-tridecynyl-2-imidazoline-1-oxide, and 1-ethoxyethyl-2-pentadecanyl-2-imidazoline-1-oxide.

Examples of suitable amine oxides represented by Formula IV, which are useful in this invention, include N-2-hydroxynonyl-morpholine oxide, N-2-hydroxypentadecyl-morpholine oxide, and N-2-hydroxyheptadecyl-morpholine oxide.

Good results are obtained when the amine oxides are used at a concentration of between 0.5% and 10% by weight of the conditioner formulation. The preferred concentration is between 1.5% and 6% of the amine oxide by weight of the hair conditioning composition.

The pH of the hair conditioning compositions is about or below the isoelectric point of hair protein. Generally speaking, the pH of the compositions of this invention are below 4.5. The pH level is dependent upon the irritating properties of the acidic conditioning composition. pH levels as low as 1.8 have been used without noticeable irritation, and it has been preliminarily observed that amine oxides, and particularly stearyl dimethyl amine oxide, act to mitigate the potential for irritation.

In the neutralizer composition embodiment of the present invention an oxidising agent is incorporated in the conditioning composition. The preferred oxidizing agent is hydrogen peroxide employed in a concentration range of 0.5 to 10% by weight, preferably 1.0 to 6%, and most preferably 1.5 to 3% by weight. Other cosmetically acceptable oxidizing agents which may be used in like concentration ranges include urea hydrogen peroxide, sodium carbonate peroxide, sodium or potassium bromate, sodium perborate or sodium hypochlorite.

In general, the composition is prepared by admixing the amine oxide, water and sufficient acid, for example hydrochloric acid, to reduce the pH to below 4.5, and preferably within the range of 2.4 - 3.8. Other acids that may be used include phosphoric acid and those organic acids (acetic, citric, glycolic) that offer sufficient acidity to accommodate the low pH range.

Other ingredients may be added to the conditioning compositions which serve known functions. For example, ethoxylated cetearyl alcohol which is an emulsifier, cetyl alcohol which is a viscosity builder and superfatting agent and other ingredients such as hydrolyzed protein, perfume, color and preservatives may be added as desired.

## General Formulation and Preparation of Conditioner Composition

(Trade marks are acknowledged as such)

| Component | %, by weight |
|---|---|
| Long Chain Alkyl Amine Oxide | 6.0 |
| Cetyl ($C_{18}H_{37}$) Alcohol with 15 moles ETO | 0.5 |
| Cetyl Alcohol | 1.0 |
| Methyl Paraben | .2 |
| Propyl Paraben | .05 |
| Hydrolyzed Animal Protein | .75 |
| FD&C Yellow #5 | .0006 |
| Fragrance Oil | .75 |
| Concentrated HCl | 1.5 |
| Water | q.s. 100 |

In the above general information cetyl-stearyl alcohol with 15 moles ETO functions as an emulsifying agent; cetyl alcohols builds creamy structure of the emulsion and provides complimentary hair conditioning; the methyl and propyl parabens are preservatives; hydrolyzed animal protein serves as a complimentary conditions and provides consumer appeal.

All the ingredients except the fragrance oil and acid are heated to 70-75° C and mixed to uniformity. The hydrochloric acid is mixed in and the preparation is slowly cooled to 60° then more quickly to 42° C. Fragrance is mixed in at 42° C and the composition is cooled to room temperature. Moderate mixing takes place throughout the cooling process.

When stearyl dimethyl amine oxide was used as the specific amine oxide in the formula the resulting product was a rich looking rheological lotion of 5.2 Pa s (5,200 cps) viscosity and pH of 2.6. When other linear amine oxides of lesser chain lengths were used, the lotions produced had lower viscosities. Additional quantities of a fatty alcohol were required in the formula to increase the viscosity of the resulting lotions. Some amine oxides such as oelyl dimethyl amine oxide, cocoamidopropyl dimethyl amine oxide, and oleyl imidazoline amine oxide produced thin lotions. It was also observed that such amine oxides were totally soluble, by themselves, in water and were capable of producing clear hair conditioning products when used in the following formulation:

| Component | %, by weight |
|---|---|
| Water Soluble Alkyl Dimethyl Amine Oxide | 6.0 |
| Hydroxyethyl Cellulose (2% Sol'n, $H_2O$) | 50.0 |
| Nonoxynol-12 | 1.0 |
| Fragrance | 1.0 |
| Concentrated HCl | 1.5 |
| Water | q.S. 100 |

The clear product has a pH of 2.6 and a viscosity of 1.5 Pa s 1,500cps). It produced sudsing when applied to hair, indicating its use as a shampoo in the application as a creme rinse conditioner after shampooing.

On the other hand, the dialkyl long chain amine oxides are more insoluble than the long chain monoalkyl amine oxides. When evaluated on hair in the above formula, the emulsion stability of the formula containing the di-long chain amine oxides was poor because of their low water solubility. A stabilized formula for a dialkyl long chain amine oxides was developed and products evaluated:

| Component | %, by weight |
|---|---|
| Di-long Chain Alkyl Methyl Amine Oxide | 1.5 |
| Polysorbate 85 | 9.0 |
| Sorbitan Trioleate | 1.8 |
| Cetyl ($C_{18}H_{37}$) Alcohol with 15 moles ETO | 2.0 |
| Cetyl Alcohol | 2.0 |
| Nonoxynol 12 | 0.5 |
| Fragrance | 0.5 |
| Concentrated HCl | q.s. pH 2.2 - 2.6 |
| Dye Blend | q.s. |
| Water | q.s. 100 |

All di-long chain amine oxides evaluated produced cosmetically elegant cremes and lotions. They all gave detangling effects on hair with the d-coco and di-stearyl giving exceptionally easy combing.

Translucent creme rinses can be formulated by combining water soluble and water insoluble amine oxides:

| Component | %, by weight |
|---|---|
| ($C_{18}H_{37}$) dimethyl amine oxide | 3.0 |
| ($C_{18}H_{35}$) dimethyl amine oxide | 3.0 |
| Fragrance | 0.6 |
| Ucon LB-1715 (PPG-40 Butyl Ether) | 0.4 |
| Hydroxyethyl Cellulose | 1.2 |
| Nonoxynol 12 (Nonylphenoxyether-12 EtO) | 0.6 |
| Concentrated HCl | 1.5 |
| Water | q.s. 100 |

This preparation was translucent, having a pH of 2.7 and a viscosity of 2.27Pa s (2,270cps). In place of stearyl dimethyl amine oxide other insoluble varieties can be used, such as the cetyl, hydrogenated tallow, dicoco amine oxide analogs. In place of oleyl dimethyl amine oxide, water soluble amine oxides may be used such as coco, cocamidopropyl, oleylimidazoline amine oxide analogs. All impart conditioning effects to hair.

The function of hydroxyethyl cellulose in these formulas is to thicken the preparation. Other thickening agents may be used such as hydroxypropyl methyl cellulose, hydroxypropyl cellulose and natural cellulose resins. "Uncon" polymers as well as nonionic block polymers of the "Pluronic" type serve to adjust viscosity. Octylphenoxy ethers and nonylphenoxy ethers serve as emulsifiers and solubilizers for the fragrance and other oils. Other emulsifying agents may be used such as ethozylated sorbitan esters (Polysorbate 65, Polysorbate 60) and polyethylene glycol ethers (PEG-400, PEG-600).

Illustrative formulations of neutralizer conditioners follow, the preparation of which is analogous to those of the condition previously described.

2% Hydrogen Peroxide Neutralizer Conditioner

| Component | %, by weight |
|---|---|
| ($C_{17}H_{35}$) amine oxide | 1.5 |
| Steareth - 10 | 1.0 |
| 50 Amino (trimethylene phosphonic acid) | 0.6 |
| 35% Hydrogen peroxide | 5.70 |
| Phosphoric acid | 2.0 |
| Water | q.s. 100 |

The creamy lotion thus obtained has a viscosity of 4.5Pa s (4,500 cps) and has a boil test peroxide

stability of 94.2%. This hair conditioner is used for neutralizing the effects of permanent waving lotion on hair and for deodorizing hair of the sulfhydryl, sulfur odor from alkali hair straightening. It renders the hair soft, supple and easily combable.

4% Hydrogen Peroxide Neutralizer Conditioner

| Component | %, by weight |
|---|---|
| Cellosize QP 100 MH (Hydroxyethyl cellulose) | 0.75 |
| Kathon CG (Isothiazolinone preservative) | 0.04 |
| 50% Hydrolyzed animal protein | 0.10 |
| ($C_{18}H_{35}$) dimethyl amine oxide | 6.0 |
| Phosphoric acid | 2.0 |
| 35% Hydrogen peroxide | 11.4 |
| 50% Amino (trimethylene phosphonic acid) | 0.6 |
| Water | q.s. 100 |

The product is clear, has a pH of 2.7 and provides neutralization of excess alkalinity, restoration of disulfide bonds, hair deodorizing effects, while also conditioning the hair.

3% Hydrogen Peroxide Neutralizer Conditioner

| Component | %, by weight |
|---|---|
| Methocel J 75 MS (Hydroxypropyl methylcellulose) | 0.5 |
| Kathon CG (Isothiazolinone preservative) | 0.04 |
| Lauryl dimethyl amine oxide | 4.0 |
| Phosphoric acid | 2.0 |
| 35% Hydrogenperoxide | 8.55 |
| 50% Amino (trimethylene phosphonic acid) | 0.6 |
| Opacifier (Styrene/Acrylate Opacifier from Morton) | 0.3 |
| Fragrance | 0.1 |
| Water | q.s. 100 |

The product is an opaque, fragranced neutralizer conditioner having a pH of 2.7.

I Evaluation of Cationic Hair Conditioning Compositions Containing An Amine Oxide

A 2-gram - 25.4 cm (10") long tress of double bleached hair is shampooed with a conventional shampoo, and reshampooed again to simulate a double shampoo typical of consumer use. The hair is rinsed thoroughly under the tap with tepid water. Five cc of a test hair conditioner is measured with a syringe and applied to the hair tress. The conditioner is worked into the hair tress for a minute and then the tress is rinsed with tepid water under the tap for one minute. The hair is touched, observed, combed, smelled and rated to a control shampoo tress without a conditioner application. Upon drying, the tress is rated again by touching, observing, combing and smelling.

Tests to determine the substantivity of the cationic amine oxide to hair protein have been conducted using the "Rubine Dye Test". The dye test for determining substantivity of cationics to hair demonstrates the degree of adhesive nature of a cationic agent to hair during rinsing with water. Hair treated with a cationic conditioner will gather a rinse-fast stain when subjected to the dye; the coloration gathered on untreatd hair is readily rinsed away. The dye complexes with positively charged surfactant residues on the hair forming a stain that resists rinsing from the hair. Pyrazole Fast Bordeau 2BL was used in these tests in place of Rubine dye because Rubine dye has become unavailable. The hair conditioning compositions containing amine oxides produce a positive Rubine Dye Test response on tresses treated using formulations described herein.

The Rubine Dye Test employed a double bleached hair tress which was treated with a cationic

conditioning product. After treatment, the tress was rinsed for exactly one minute under tepid tap water. The tress was then towel-dried and immersed into a 0.2% aqueous Pyrazol Fast Bordeau 2BL dye solution for 10 seconds. Again, the tress was rinsed under the tap to remove excess dye solution from hair. A residual red stain left on the hair indicated a substantive deposition of cationic amine oxide, whereas a free-rinsed control hair tress that had not been treated with the cationic conditioner prior to treatment with dye did not retain a red stain.

The hair conditioning delivered by the amine oxide compositions of this invention have properties that are variable and that which may be adjusted for in formulation. Since conditioning effects are relative to the needs of the user, it is a convenience to have adjustable features in formula development to suit the formulator's objectives. Certain users prefer to have as their major objective in hair conditioning excellent detangling of shampooed hair. Others prefer to have less detangling effectiveness but required that their hair feel natural, not overconditioned or heavily coated. Some users like to use clear products; others opaque cremes and lotions. Most users prefer to have their hair free of static charge to allow good manageability. The wide range of physical properties that various amine oxides offer are taken advantage of at or about the isoelectric point of hair protein to produce tailor-made products that have features that satisfy the user.

As a corollary, it is difficult to measure the attributes of a hair conditioning product with only one parameter describing conditioning. In the evaluation of amine oxides and their formulations three parameters have been used to assess hair conditioning effects:

1) The Rubine Dye Test serves to demonstrate the substantivity of cationic ingredients in hair conditioners. The substantive coating, that shows red with Rubine dye, is composed of positive charged and/or polarized molecules which tend to conduct ions or electrons (the localized accumulation of such ions or electrons is the cause of static charges). A positive Rubine Dye Test, therefore indicates that because of the substantive coating on the hair which is conductive, any accumulating ions or electrons will be mobile and any electrostatic charges therefrom are readily dissipated. Hence, the disadvantages to manageability of hair from static electricity are nullified.

2) Touching hair serves for inform the user the state of conditioning in one's hair. The feeling is totally subjective, varying among individuals according to personal preferences. Some prefer light texture, approaching a natural or unconditioned effect; others prefer the tactile demonstration of conditioning provided by a significant coating of fatty material. In the laboratory evaluation of the "touch" parameter, using a range of 1 to 10, 10 signified a clean feeling, the absence of coating which is apparently present (Rubine Dye Test) and which can offer other advantages; 1 signified a maximum, heavily conditioned coating that can be felt with the fingers. Either effects, a clean feeling or a definitive "conditioned" coating, can be desirable depending on the users perspective.

3) The ease rendered in combing wet hair after shampooing is perhaps the single most important benefit of creme rinse products. Immediately after shampooing hair is usually left matted and difficult to comb through. Damage to the hair structure usually results upon combing or brushing at this stage because of the intense friction produced on the tangled hair. Furthermore, pulling and stretching the hair during wet combing results in the weakening of its tensile strength, some degree of hair breakage and causes pain and discomfort to the individual. The application of a creme rinse balsam or other hair conditioning treatment provides a lubricating coating to the hair shaft that reduces and minimizes the combing effort. The user is thus spared the discomfort of combing tangled and snarled hair. In laboratory evaluation, the effectiveness of a conditioner application in providing easy combing after a shampoo treatment is rated on a 1 to 10 scale. A rating of 10 indicates easy wet combing comparable to the effects of a leading commercial hair conditioner based upon quaternary ammonium surfactants; a rating of 1 indicates the base state of combing hair after shampooing with a detergent cleanser and without a hair conditions application.

Cationic hair conditioning formulations were made as described above and tested. The results of the tests are shown in the table below.

| Conditioning Agents | Rubine Dye Test | Touch Parameter | Hair Detangling Effectiveness |
|---|---|---|---|
| Lauryl dimethyl amine oxide | + | 10 | 2 |
| ($C_{18}$) Hydrocarbon amido propyl dimethyl amine oxide | + | 9.5 | 5 |
| ($C_{14}H_{29}$) dimethyl amine oxide | + | 9 | 4 |
| ($C_{18}H_{37}$) dimethyl amine oxide | + | 7 | 10 |
| Cetyl dimethyl amine oxide | + | 8 | 8 |
| ($C_{18}H_{35}$) dimethyl amine oxide | + | 10 | 5 |
| ($C_{17}$-$C_{19}$) dimethyl amine oxide | + | 7.5 | 6 |
| ($C_{18}$) hydrocarbon amine dimethyl amine oxide | + | 9.5 | 3 |
| ($C_{18}$) hydrocarbon bis (hydroxyethyl) amine oxide | + | 10 | 3 |
| Tallow bis (hydroxyethyl) amine oxide | + | 7 | 3 |
| ($C_{18}H_{35}$) imidazoline amine oxide | + | 10 | 3 |
| Quaternary ammonium salt (Commercial Product Control) | + | 3 | 10 |

II. Evaluation of Cationic Hair Conditioning Compositions Containing an Amine Oxide and an Oxidizing Agent

A formulation of the present invention was evaluated be treating the hair of three panelists therewith. The tests were conducted by sectioning off hair on half the scalp of each panelist after a standard permanent wave lotion containing a reducing agent was applied thereto. After thoroughly rinsing the permanent waving lotion from the hair and towel drying, one of the hair sections of each panelist was treated with the test formulation; the other section with an established commercial neutralizer of conventional formulation. After about five minutes the hair of both sections was rinsed freely under the tap.

Two of the three panelists tested preferred the oxidative conditioning neutralizer of the present invention over the control for the reason that it provided the hair with a smoother slip, tighter curl and an overall improved conditioning effect. The third panelist found the control and test product treated hair sections about equal.

**Claims**

**1.** Use of an amine oxide as sole conditioning agent in a neutralizer conditioning composition comprising:
a) 0.1 to 10 % by weight of a cosmetically acceptable oxidizing agent,
b) water,
c) sufficient acid to provide a pH for the composition between 4.5 and 1.8, and
d) an amine oxide acquiring a sufficient positive charge to allow the amine oxide to be substantive onto the hair, said amine oxide having a concentration from 0.1 % to 10 % of the total weight of the neutralizer conditioning composition, the amine oxide having a general formula selected from:

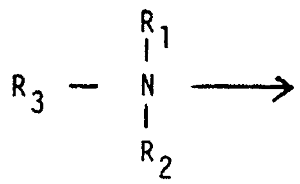
(I)

wherein $R_1$ is lower ($C_1$-$C_4$) alkyl, alkoxyalkyl or hydroxyalkyl, $R_2$ is lower to long chain ($C_1$-$C_{22}$) aliphatic hydrocarbyl radical, alkoxyalkyl or hydroxyalkyl, and $R_3$ is aliphatic hydrocarbyl moiety of 8 to 22 carbon atoms;

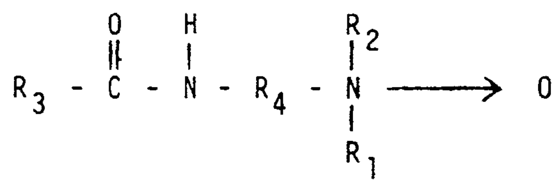
(II)

wherein $R_1$ and $R_2$ are the same or different moieties and are selected from lower ($C_1$-$C_4$) alkyl, alkoxyalkyl or hydroxyalkyl groups, $R_3$ is a moiety containing an alkyl chain of 8 to 22 carbon atoms, and $R_4$ is a moiety selected from lower ($C_1$-$C_4$) alkylene groups;

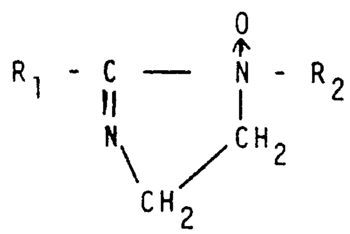
(III)

wherein $R_1$ is a moiety having an alkyl chain of 8 to 22 carbon atoms, and $R_2$ is a moiety selected from 2-hydroxyethyl, a derivative of 2-hydroxyethyl, and a nonionic derivative of 2-amino ethyl;

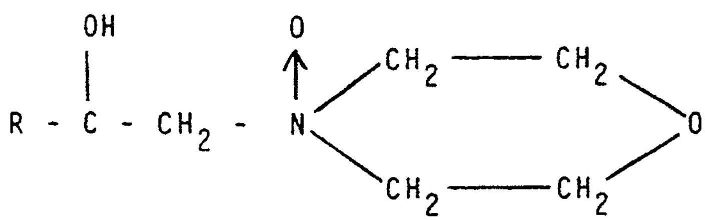
(IV)

wherein R is a moiety having an alkyl chain of 6 to 17 carbon atoms; or

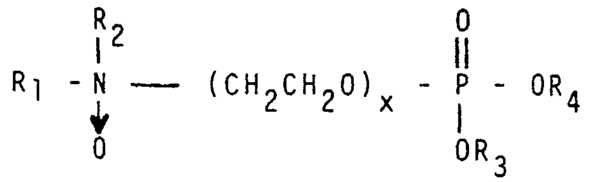

(V)

wherein $R_1$ is a moiety that contains an alkyl chain having 8 to 22 carbon atoms; $R_2$ is a moiety selected from

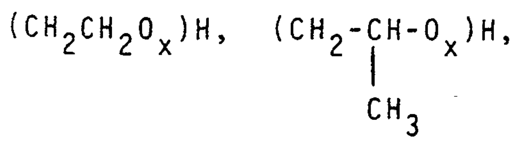

and
an alkyl chain of 8 to 22 carbon atoms, x is an integer from 1 to 30, and $R_3$ and $R_4$ are the same or different moieties and are selected from a lower ($C_1$-$C_4$) alkyl group.

**2.** Use of an amine oxide as sole conditioning agent in a neutralizer conditioning composition according to claim 1, wherein said oxidizing agent is urea hydrogen peroxide, sodium carbonate peroxide, sodium bromate, potassium bromate, sodium perborate and sodium hypochlorite or hydrogen peroxide.

**3.** Use of an amine oxide as sole conditioning agent in a neutralizer conditioning composition according to claim 1, wherein the oxidizing agent concentration is from 0.5 to 10 % based on the total weight of said neutralizer conditioning composition.

**4.** Use of an amine oxide as sole conditioning agent in a neutralizer conditioning composition according to any of claims 1 to 3, wherein the composition has a pH from 3.8 to 2.4 and the amine oxide concentration is from 1 % to 6 % based on the total weight of said neutralizer conditioning composition.

**5.** Use of an amine oxide as sole conditioning agent in a neutralizer conditioning composition according to any of claims 1 to 4, wherein the acid to provide a pH for the composition of or below the isoelectric pH of hair protein is phosphoric acid.

**6.** Use of an amine oxide as sole conditioning agent in a neutralizer conditioning composition according to any of claims 1 to 5, wherein the oxidizing agent is hydrogen peroxide.

**7.** Use of an amine oxide as sole conditioning agent in a neutralizer conditioning composition according to any of claims 1 to 6, wherein the pH is from 2.4 to 4.5.

**8.** Use of an amine oxide as sole conditioning agent in a neutralizer conditioning composition according to any of claims 1 to 7, wherein the amine oxide is ($c_{18}H_{37}$) dimethyl amine oxide, di($C_{18}H_{37}$) monomethyl amine oxide, ($c_{18}H_{37}$) aminopropyl dimethyl amine oxide, ($c_{18}H_{35}$) imidazoline amine oxide, ($C_{18}H_{37}$) dimethyl amine oxide or lauryl dimethyl amine oxide.

## Revendications

**1.** Utilisation d'un oxyde d'amine comme unique agent de conditionnement dans une composition de conditionnement neutralisante comprenant :

a) 0,1 à 10% en poids d'un agent oxydant cosmétiquement acceptable ;
b) de l'eau ;
c) suffisamment d'acide pour procurer à la composition un pH se situant entre 4,5 et 1,8 ; et
d) un oxyde d'amine acquérant une charge positive suffisante pour permettre à l'oxyde d'amine d'avoir une affinité spécifique pour les cheveux, ledit oxyde d'amine ayant une concentration allant de 0,1% à 10% du poids total de la composition de conditionnement neutralisante, l'oxyde d'amine

ayant une formule générale choisie parmi :

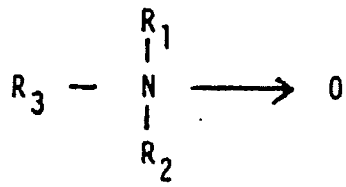

(I)

dans laquelle :

- $R_1$ représente alkyle inférieur ($C_1$-$C_4$), alcoxyalkyle ou hydroxyalkyle ;
- $R_2$ représente un radical hydrocarbyle aliphatique allant d'inférieur jusqu'à longue chaîne ($C_1$-$C_{22}$), alcoxyalkyle ou hydroxyalkyle ; et
- $R_3$ représente une fraction hydrocarbyle aliphatique de 8 à 22 atomes de carbone ;

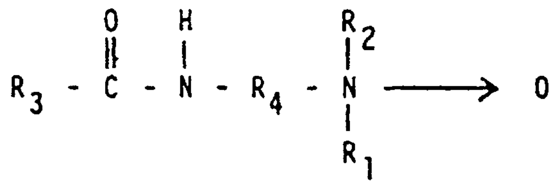

(II)

dans laquelle :

- $R_1$ et $R_2$ représentent des fractions identiques ou différentes et sont choisies parmi les groupes alkyle inférieurs ($C_1$-$C_4$), alcoxyalkyle ou hydroxyalkyle ;
- $R_3$ représente une fraction contenant une chaîne alkyle de 8 à 22 atomes de carbone ; et
- $R_4$ représente une fraction choisie parmi les groupes alkylène inférieurs ($C_1$-$C_4$) ;

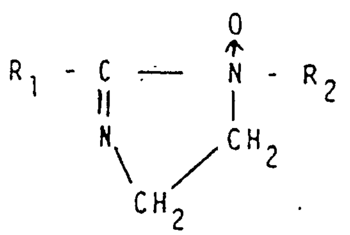

(III)

dans laquelle :

- $R_1$ représente une fraction ayant une chaîne alkyle de 8 à 22 atomes de carbone ; et
- $R_2$ représente une fraction choisie parmi hydroxy-2 éthyle, un dérivé d'hydroxy-2 éthyle, et un dérivé non-ionique d'amino-2 éthyle ;

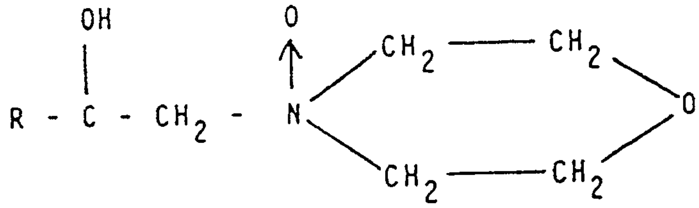

(IV)

dans laquelle :

- R représente une fraction ayant une chaîne alkyle de 6 à 17 atomes de carbone ; ou

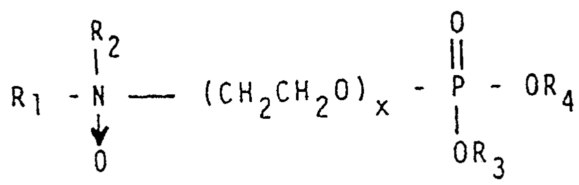 (V)

dans laquelle :

- $R_1$ représente une fraction qui contient une chaîne alkyle ayant 8 à 22 atomes de carbone ;
- $R_2$ représente une fraction choisie parmi

  $(CH_2CH_2O_x)H$, 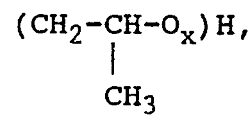

  et

  une chaîne alkyle de 8 à 22 atomes de carbone ;
- x est un nombre entier de 1 à 30 ; et
- $R_3$ et $R_4$ sont des fractions identiques ou différentes et sont choisies parmi les groupes alkyle inférieurs ($C_1$-$C_4$).

2. Utilisation d'un oxyde d'amine comme unique agent de conditionnement dans une composition de conditionnement neutralisante selon la revendication 1, dans laquelle ledit agent oxydant est l'urée peroxyde d'hydrogène, le peroxyde de carbonate de sodium, le bromate de sodium, le bromate de potassium, le perborate de sodium et l'hypochlorite de sodium ou le peroxyde d'hydrogène.

3. Utilisation d'un oxyde d'amine comme unique agent de conditionnement dans une composition de conditionnement neutralisante selon la revendication 1, dans laquelle la concentration de l'agent oxydant va de 0,5 à 10% sur la base du poids total de ladite composition de conditionnement neutralisante.

4. Utilisation d'un oxyde d'amine comme unique agent de conditionnement dans une composition de conditionnement neutralisante telle que définie à l'une des revendications 1 à 3, dans laquelle la composition a un pH allant de 3,8 à 2,4 et la concentration de l'oxyde d'amine va de 1% à 6% sur la base du poids total de ladite composition de conditionnement neutralisante.

5. Utilisation d'un oxyde d'amine comme unique agent de conditionnement dans une composition de conditionnement neutralisante telle que définie à l'une des revendications 1 à 4, dans laquelle l'acide destiné à procurer à la composition un pH égal au, ou au-dessous du, pH isoélectrique de la protéine du cheveu, est l'acide phosphorique.

6. Utilisation d'un oxyde d'amine comme unique agent de conditionnement dans une composition de conditionnement neutralisante telle que définie à l'une des revendications 1 à 5, dans laquelle l'agent oxydant est l'eau oxygénée.

7. Utilisation d'un oxyde d'amine comme unique agent de conditionnement dans une composition de conditionnement neutralisante telle que définie à l'une des revendications 1 à 6, dans laquelle le pH va de 2,4 à 4,5.

8. Utilisation d'un oxyde d'amine comme unique agent de conditionnement dans une composition de conditionnement neutralisante telle que définie à l'une des revendications 1 à 7, dans laquelle l'oxyde d'amine est l'oxyde de ($C_{18}$-$H_{37}$) diméthyl amine, l'oxyde de di($C_{18}H_{37}$) monométhyl amine, l'oxyde de ($C_{18}H_{37}$) aminopropyl diméthyl amine, l'oxyde de ($C_{18}H_{35}$) imidazoline amine, l'oxyde de ($C_{18}H_{37}$) diméthyl amine ou l'oxyde de lauryl diméthyl amine.

**Patentansprüche**

**1.** Verwendung eines Aminoxids als einziges Konditionierungsmittel in einer neutralisierenden Konditionierungszusammensetzung beinhaltend:

a) 0,1-10 Gew.-% eines kosmetisch akzeptablen Oxidationsmittels,

b) Wasser

c) ausreichend Säure um einen pH-Wert der Zusammensetzung zwischen 4,5 und 1,8 zu erreichen und

d) ein Aminoxid, das eine ausreichende positive Ladung erreicht, um tatsächlich auf dem Haar zu wirken, mit einer Konzentration des Aminoxids von 0,1 % bis 10 % des gesamten Gewichts der neutralisierenden Konditionierungszusammensetzung, wobei das Aminoxid eine allgemeine Formel hat, ausgewählt aus:

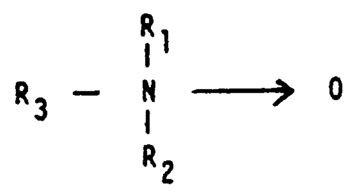

(I)

wobei $R_1$ ein niedriges ($C_1$-$C_4$) Alkyl, Alkoxyalkyl oder Hydroxyalkyl ist, $R_2$ eine kurze bis lange Kette ($C_1$-$C_{22}$) eines aliphatischen Kohlenwasserstoffrestes, Alkoxyalkyl oder Hydroxyalkyl ist und $R_3$ ein aliphatischer Kohlenwasserstoff mit einem Anteil von 8 bis 22 Kohlenstoffatomen ist;

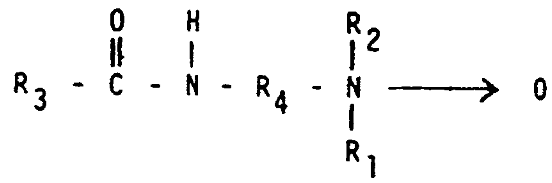

(II)

wobei $R_1$ und $R_2$ gleiche oder verschiedene Teile und ausgewählt sind aus niederen ($C_1$-$C_4$)-Alkyl-, Alkoxyalkyl- oder Hydroxyalkylgruppen, $R_3$ ein Teil enthaltend eine Alkylkette mit 8 bis 22 Kohlenstoffatomen ist und $R_4$ ein Teil ausgewählt aus niedrigen ($C_1$-$C_4$)-Alkylengruppen ist;

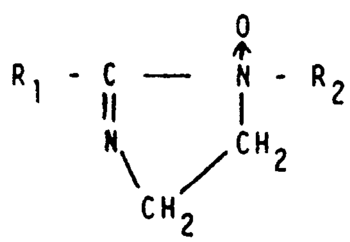

(III)

wobei $R_1$ ein Teil mit einer Alkylkette mit 8 bis 22 Kohlenstoffatomen ist und $R_2$ ein Teil ausgewählt aus 2-Hydroxyethyl, einem Derivat von 2-Hydroxyethyl und einem nichtionischen Derivat von 2-Aminoethyl ist;

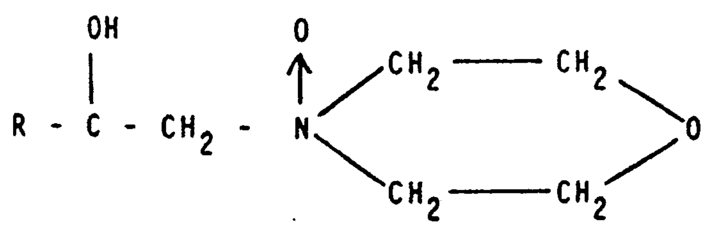

(IV)

wobei R ein Teil mit einer Alkylkette von 6 bis 17 Kohlenstoffatomen ist; oder

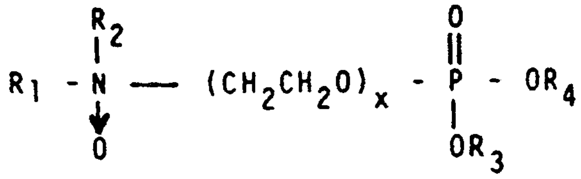 (V)

wobei $R_1$ ein Teil enthaltend eine Alkylkette mit 8 bis 22 Kohlenstoffatomen ist; $R_2$ ein Teil ist ausgewählt aus

$(CH_2CH_2O_x)H$, 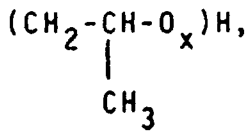

und
einer Alkylkette mit 8 bis 22 Kohlenstoffatomen, x eine ganze Zahl von 1 bis 30 ist und $R_3$ und $R_4$ gleiche oder verschiedene Teile und ausgewählt aus niedrigen $(C_1$-$C_4)$Alkylgruppensind.

2. Verwendung eines Aminoxids als einziges Konditionierungsmittel in einer neutralisierenden Konditionierungszusammensetzung nach Anspruch 1, wobei das Oxidationsmittel Harnstoffwasserstoffperoxid, Natriumcarbonatperoxid, Natriumbromat, Kaliumbromat, Natriumperborat und Natriumhyperchlorid oder Wasserstoffperoxid ist.

3. Verwendung eines Aminoxids als einziges Konditionierungsmittel in einer neutralisierenden Konditionierungszusammensetzung nach Anspruch 1, wobei die Konzentration des Oxidationsmittels von 0,5 bis 10 % basierend auf dem Gesamtgewicht der neutralisierenden Neutralisierungszusammensetzung ist.

4. Verwendung eines Aminoxids als einziges Konditionierungsmittel in einer neutralisierenden Konditionierungszusammensetzung nach einem der Ansprüche 1 bis 3, wobei das Mittel einen pH-Wert von 3,8 bis 2,4 hat und die Aminoxidkonzentration von 1 bis 6 % basierend auf dem Gesamtgewicht der neutralisierenden Konditionierungszusammensetzung ist.

5. Verwendung eines Aminoxids als einziges Konditionierungsmittel in einer neutralisierenden Konditionierungszusammensetzung nach einem der Ansprüche 1 bis 4, wobei die Säure, die einen pH-Wert für die Zusammensetzung liefert, der gleich oder unterhalb des isoelektrischen pH-Werts der Haarproteine ist, Phosphorsäure ist.

6. Verwendung eines Aminoxids als einziges Konditionierungsmittel in einer neutralisierenden Konditionierungszusammensetzung nach einem der Ansprüche 1 bis 5, wobei das Oxidationsmittel Wasserstoffperoxid ist.

7. Verwendung eines Aminoxids als einziges Konditionierungsmittel in einer neutralisierenden Konditionierungszusammensetzung nach einem der Ansprüche 1 bis 6, wobei der pH-Wert von 2,4 bis 4,5 beträgt.

8. Verwendung eines Aminoxids als einziges Konditionierungsmittel in einer neutralisierenden Konditionierungszusammensetzung nach einem der Ansprüche 1 bis 7, wobei das Aminoxid $(C_{18}H_{37})$-Dimethylaminoxid, Di$(C_{18}H_{37})$monoethylaminoxid, $(C_{18}H_{37})$-Aminopropyldimethylaminoxid, $(C_{18}H_{35})$-Imidazolinaminoxid, $(C_{18}H_{37})$-Dimethylaminoxid oder Lauryldimethylaminoxid ist.